# EUROPEAN PATENT APPLICATION

(11) **EP 2 453 017 A1**
(43) Date of publication of application: **16.05.2012**
(21) Application number: 10191103.0
(22) Date of filing: 12.11.2010
(51) Int. Cl.: C12N 15/113, A61K 31/7088, A61P 35/00

(54) **Oligonucleotides for use in prophylaxis and/or treatment of TGF-beta1 and TGF-beta2, TGF-beta2 and TGF-beta3, TGF-beta1 and TGF-beta3, or TGF-beta1, TGF-beta2, and TGF-beta3 mRNA overexpressing diseases**

(71) Applicant: Antisense Pharma GmbH, 93053 Regensburg (DE)
(72) Inventor: Jaschinski, Frank, 93083 Obertraubling (DE); Rothammer-Hampl, Tanja, 93138 Lappersdorf (DE); Schneider, Anneliese, 82340 Feldafing (DE); Schlingensiepen, Karl-Hermann, 93055 Regensburg (DE)
(74) Representative: Jansen, Cornelis Marinus

(57) **Abstract**

The present invention refers to an oligonucleotide for use in prophylaxis and/or treatment of a TGF-beta1 and TGF-beta 2 mRNA overexpressing tumor, a TGF-beta2 and TGF-beta3 mRNA overexpressing tumor, a TGF-beta1 and TGF-beta3 mRNA overexpressing tumor, or a TGF-beta1, TGF-beta 2, and TGF-beta3 mRNA overexpressing tumor.

## Description

The present invention refers to an oligonucleotide for use in prophylaxis and/or treatment of a TGF-beta1 and TGF-beta2, TGF-beta2 and TGF-beta3, TGF-beta1 and TGF-beta3, or TGF-beta1, TGF-beta2, and TGF-beta3 mRNA overexpressing disease, preferably a tumor, wherein one type of oligonucleotide reduces or inhibits the expression of at least two TGF-beta isoforms. Further, the invention is directed to a pharmaceutical composition comprising such oligonucleotide, and a method for the preparation of such pharmaceutical composition.

Transforming growth factor beta (TGF-beta) is involved in numerous diseases and plays a key role in regulating cancer pathomechanisms. Three mammalian isoforms of TGF-beta have been isolated yet, which are TGF-beta1, TGF-beta2, and TGF-beta 3, and which are differently expressed in different diseases. For example only one isoform is increased, two or all three isoforms are increased, or one or more isoforms are decreased, or one or more isoforms are increased and at the same time another TGF-beta isoform or isoforms are decreased.

### Figures

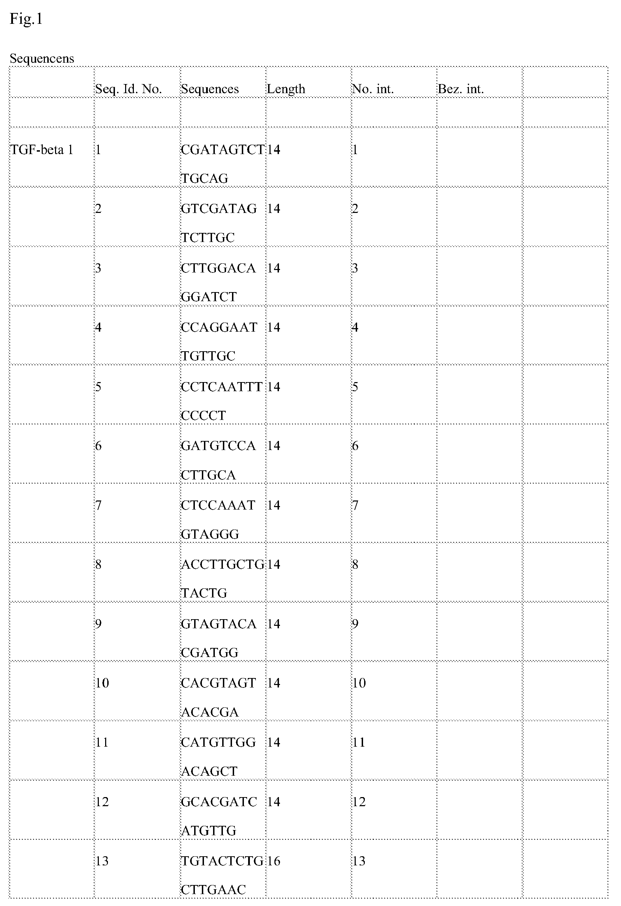

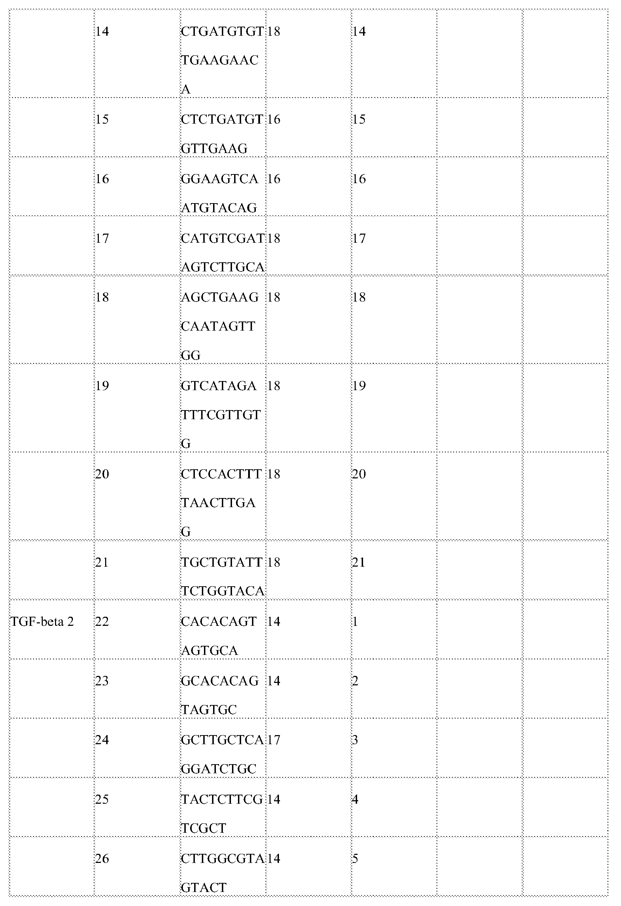

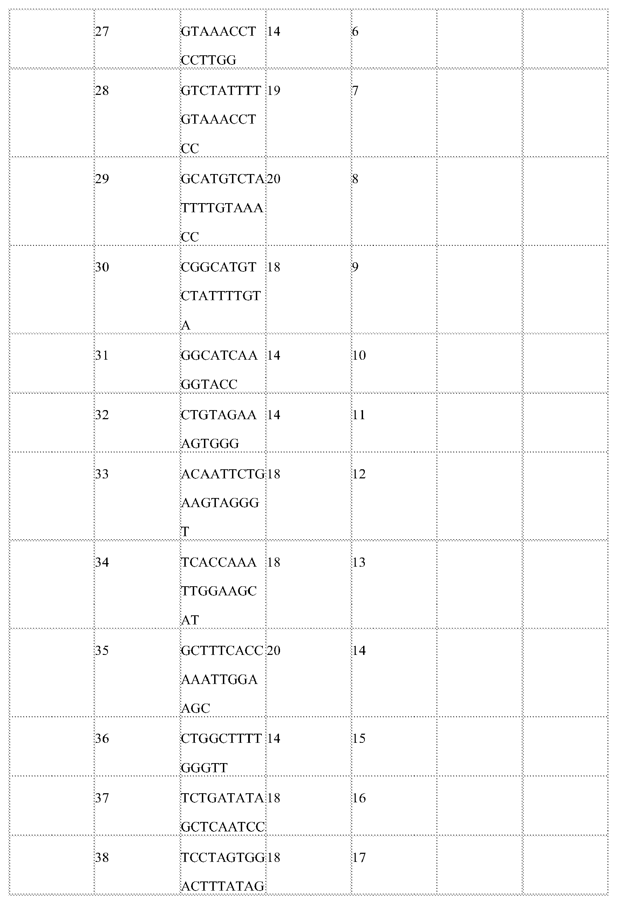

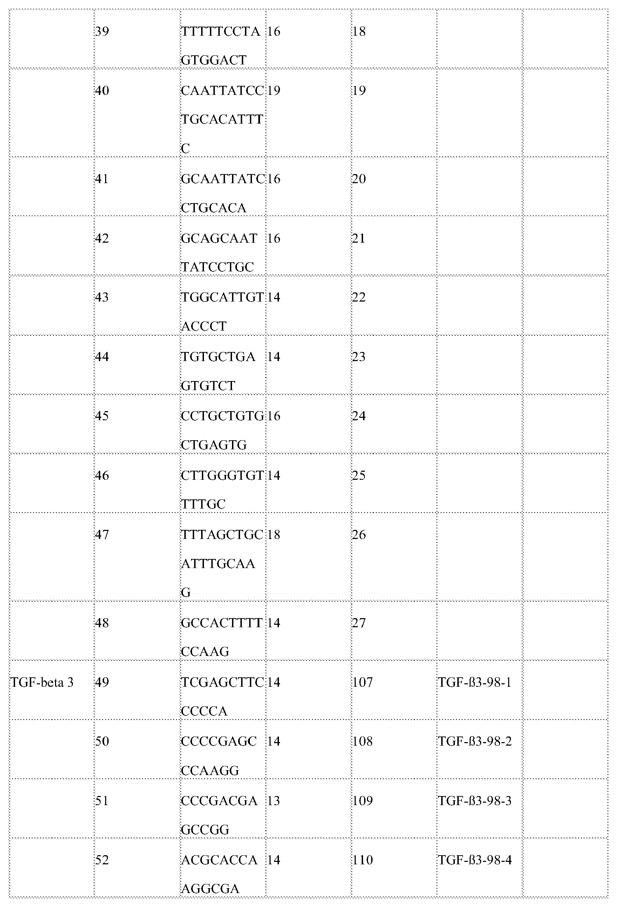

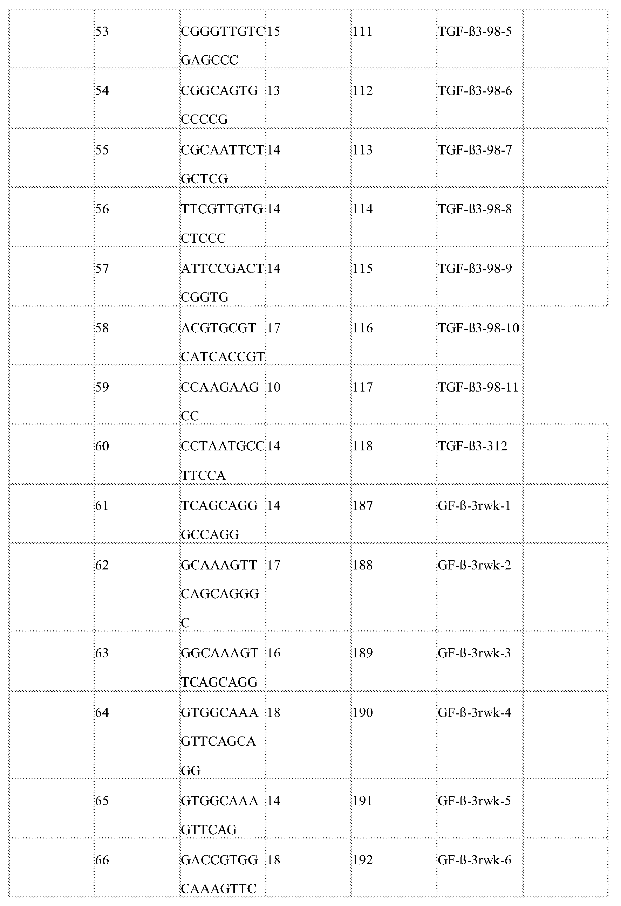

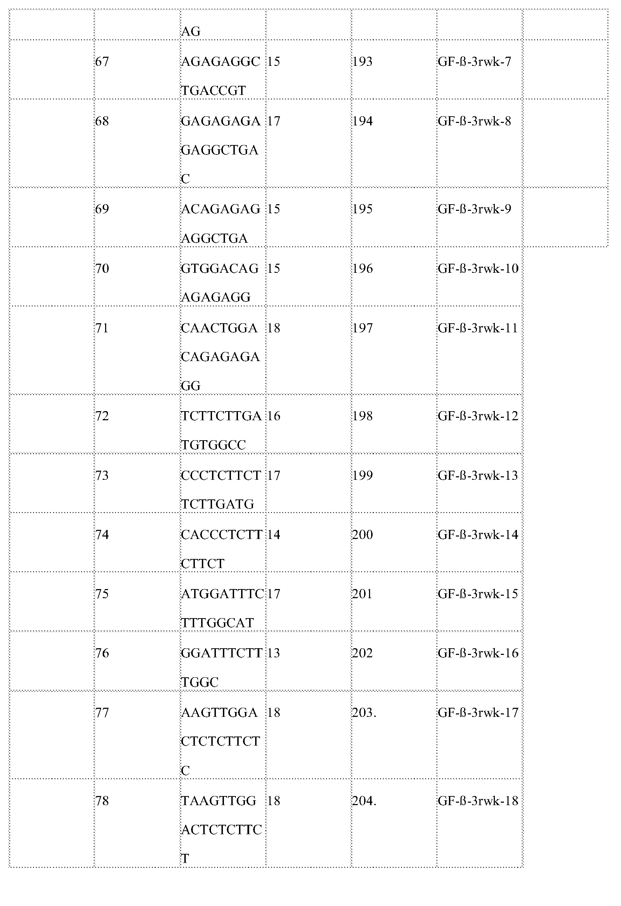

Fig. 1 shows SEQ ID No. 1 to 78, which hybridize with TGF-beta1, TGF-beta2, and/or TGF-beta3.
Fig. 2 presents the inhibition of TGF-beta1 and TGF-beta2 expression in A-172 human glioma cells by adding an antisense oligonucleotide for example of SEQ ID No. 30.

The present invention shows that an oligonucleotide, for example a TGF-beta2 antisense oligonucleotide, reduces or inhibits overexpressed TGF-beta1 and TGF-beta2 mRNA, TGF-beta2 and TGF-beta3, TGF-beta1 and TGF-beta3, or TGF-beta1 TGF-beta2, and TGF-beta3 mRNA. The great advantage of the present invention is that one type of oligonucleotide reduces or inhibits the expression of at least two different TGF-beta isoforms, and thus, is highly efficient for use in the prophylaxis and/or treatment of TGF-beta involved diseases. Such prophylaxis and/or treatment refer, beside tumors, for example to corneal regenerative wound repair, keloid scar formation, bone formation, ischemia, renal tubulointerstitial fibrosis, renal fibrogenesis, scars, etc.

Tumors which are sensitive to the use of an oligonucleotide in prophylaxis and/or treatment are for example selected from the group consisting of glioma (e.g., glioblastoma multiforme), astrocytoma (e.g., astrocytoma malignum), pancreatic carcinoma (e.g., ductal adenocarcinoma), prostate carcinoma, lung carcinoma (e.g., non-small cell lung carcinoma (NSCLC), squamous cell lung cancer, adenocarcinoma, large cell lung cancer, small cell lung cancer (SCLC)), skin cancer (e.g., squamous cell carcinoma, basal cell carcinoma, malignant melanoma), kidney cancer (e.g., renal cell carcinoma, pheochromocytoma, urothelial cell carcinoma), renal carcinoma, ovary cancer (e.g., adenocarcinoma, ovarian epithelial carcinoma, mucinous cystadenocarcinoma), breast cancer (e.g, ductal carcinoma, lobular carcinoma), esophageal cancer (e.g., squamous cell carcinoma, adenocarcinoma), stomach cancer (e.g., adenocarcinoma), bladder cancer, colon cancer (e.g., adenocarcinoma, squamous epithelium carcinoma), colorectal cancer, gastric cancer, nasopharynx or larynx cancer (e.g., squamous cell carcinoma, lymphoepithelioma), thyreoid gland cancer (e.g., of papillary thyroid cancer, follicular thyroid cancer, medullary thyroid cancer, anaplastic thyreoid cancer), bone cancer (e.g., osteosarcoma, chondrosarcoma), pleura cancer (e.g., mesothelioma), liver cancer (e.g., heptocellular carcinoma), uterine cancer (e.g., endometrial cancer, squamous cell carcinoma), or endometrial cancer, in particular in a late stage and/or if the tumor has high TGF-beta-Smad activity.

Further tumors are carcinoma embronal, choriocarcinoma, teratoma, Hodgkin's Lymphoma (NHL), or leukemia, e.g., acute or myeloblastic leukemia.

In particular, an oligonucleotide of the present invention is highly efficient in reduction or inhibition of TGF-beta1 and TGF-beta2, TGF-beta2 and TGF-beta3, TGF-beta1 and TGF-beta3, or TGF-beta1, TGF-beta2, and TGF-beta3 mRNA overexpression in PC-3 cells (prostate), A-549 cells (NSCLC), pediatric glioma, HTZ-349 (high grade glioma), A-172 (glioma), PANC-1 cells (pancreas), PA-TU-8902 cells (pancreas), L3.6pL cells (pancreas), CAKI (renal cell), DU-145 (prostate), Mel-Juso (melanoma), C8161 (melanoma), KYSE-140 (esophagus), or NCI-H661 (lung). These cells are equivalent to above mentioned tumors, i.e., they were isolated from these tumors and form the basis for a cell line.

In a further embodiment, the oliognucleotide is used in reduction or inhibition of TGF-beta1 and TGF-beta2, TGF-beta2 and TGF-beta3, TGF-beta1 and TGF-beta3, or TGF-beta1, TGF-beta2, and TGF-beta3 mRNA overexpression in a treatment resistant tumor cells. The resistance of the tumor cell refers for example to chemotherapeutic resistance, resistance against radiation, or resistance against targeted therapies like resistance against an antibody, a small molecule, etc. The treatment results in a decrease of the resistance and in an increase of the sensitivity of the tumor cell against the chemotherapeutic.

The oligonucleotide of the present invention preferably hybridizes with mRNA of TGF-beta1, TGF-beta-2, or TGF-beta3, for example the oligonucleotide consists of or comprises one of SEQ ID No. 1 to 21, 22 to 48, or 49 to 78.

The oligonucleotide of this invention has a length of about 8 to about 30 nucleotide building blocks, and include oligonucleotides having non-naturally occurring portions with similar function. Naturally occurring nucleotides as well as non- naturally occurring nucleotides, modifications and spacers are also referred to as nucleotide building block. The most common nucleotide building block is a nucleotide. Modified or substituted nucleotides as well as spacers are also comprised by the expression nucleotide building block.

These modified or substituted oligonucleotides are often preferred over native forms because of desirable properties such as enhanced cellular uptake, enhanced affinity for nucleic acid target (e.g. protein), altered intracellular localization and increased stability in the presence of nucleases. Modifications of the oligonucleotides as used herein comprise any chemical modification of the sugar, the base moiety and/or the internucleotide linkage.

In one embodiment, the ring structure of the ribose group of the nucleotides in the modified oligonucleotide or polynucleotide has oxygen in the ring structure substituted with N-H, N-R (with R being an alkyl, more preferred alkyl with 1 to 20 carbons, very preferred alkyls are methyl, ethyl, propyl, isopropyl, butyl, isobutyl, or R is an aryl substituent), S and/or methylene.

In one embodiment, nucleic acids or oligonucleotides with a covalently modified base and/or sugar include for example nucleic acids having backbone sugars, which are covalently attached to low molecular weight organic groups other than a hydroxyl group at the 3' and/or 2' position or a phosphate group at the 5' position. Thus, modified nucleic acids may additionally include at least one 2'-O-substituted ribose group. For purposes of this invention, the term "2 '-substituted" means substitution of the 2'-OH of the ribose molecule. The O may be substituted with N, S and the substituent can further comprise an alkyl with 1 to 20 carbons, e.g. O-alkyl, S-alkyl, NH-alkyl, N-dialkyl, O- aryl, S-aryl, NH-aryl, O-aralkyl, S-aralkyl, and NH-aralkyl. Preferred embodiments of 2'-O-alkyl-groups are methoxy-, ethoxy-, propyloxy-, isopropyloxy-, and methoxy- ethoxy. Further modifications of nucleotide building blocks are given by patents US 6,143,881, US 5,591,721, US 5,652,355, US 5,962,425, US 5,969,116 and US 5,914,396. Another preferred embodiment is a nucleotide building block, comprising at least one deoxyribose with an alkyl-group bound to the 2'-carbon. The alkyl may have about 1 to about 30 carbons, 1 to about 20 carbons, 1 to about 10 carbons, or 1 to about 5 carbons. Preferred alkyl-groups are ethyl-, propyl-, isopropyl-, butyl-group, highly preferred is the methyl-group.

In yet another embodiment, modified nucleic acids include sugars such as arabinose instead of ribose. Thus the nucleic acids may be heterogeneous in backbone composition, thereby containing any possible combination of polymer units linked together such as peptide-nucleic acids (which have amino acid backbone linked to nucleic acid bases). In some embodiments the nucleic acids are homogeneous in backbone composition. The substituted purines and pyrimidines of the nucleic acids include standard purines and pyrimidines such as cytosine as well as base analogs such as substituted bases (Wagner et al. 1993). Purines and pyrimidines include, but are not limited to adenine, cytosine, guanine, thymine, inosin, 5-methylcytosine, 2-aminopurine, 2-amino-6- chloropurine, 2,6-diaminopurine, hypoxanthine, and other naturally and non-naturally occurring nucleobases, substituted and unsubstituted aromatic moieties.

The single nucleotides in each oligonucleotide may contain the same modifications, may contain combinations of these modifications, or may combine these modifications with phosphodiester linkages. Methods of rendering oligonucleotide nuclease resistant include, but are not limited to, covalently modifying the purine or pyrimidine bases. For example, bases may be methylated, hydroxymethylated, or otherwise substituted (e.g., glycosylated) such that the oligonucleotides or polynucleotides are rendered substantially acid and nuclease resistant. In a preferred embodiment, at least one end of the oligonucleotide is a biotin, biotin analog, avidin, or avidin analog. These molecules have the ability to block the degradation of the protected oligonucleotide and provide means for high affinity attachment of the modified nucleic acids to the solid support. Avidin and biotin derivatives, which can be used to prepare the reagents of this invention, include streptavidin, succinylated avidin, monomeric avidin, biocytin (biotin-epsilon-N-lysine), biocytin hydrazide, amine or sulfhydryl derivatives of 2-iminobiotin and biotinyl-epsilon-aminocaproic acid hydrazide. Additional biotin derivatives, such as biotin-N-hydroxysuccinimide ester, biotinyl-epsilon-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)-biocytin, can also be used as end-blocking groups on the oligonucleotides of the present invention.

In a further preferred embodiment, the oligonucleotide is conjugated with a polymer, in particular PEG, at the 3'- and/or 5 '-end of the oligonucleotide and/or at any internal nucleotide of the oligonucleotide as described in WO 2008/077956 A2.

In an alternative embodiment the oligonucleotide is an aptamer or a gapmer interacting with a DNA or RNA sequence.

In yet another embodiment, the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, US 5,539,082, US 5,714,331, and US 5,719,262. Further teaching of PNA compounds can be found in Nielsen et al. 1991.

Further backbone modified oligonucleotide includes, for example, phosphorothioates, chiral phosphorothioates, phosphorodithioates, phosphorotriesters, aminoalkyl-phosphorotriesters, methyl- and other alkyl-phosphonates including 3'-alkylene phosphonates and chiral phosphonates, phosphinates, phosphoramidates, including 3'-aminophosphoramidate and aminoalkylphosphoramidates, thiono-phosphor-amidates, thionoalkylphosphonates, thionoalkylphosphotriesters, and boranophosphates having normal 3 '-5 linkages, 2 '-5 linked analogs of these, and those having inverted polarity, wherein the adjacent pairs of nucleoside units are linked 3 '-5 'to 5 '-3 'or 2 '-5 'to 5 '-2'. Various salts, mixed salts, and free acid forms are also included. One preferred embodiment is the sodium salt of the nucleic acid.

In some embodiments at least one nucleotide of an oligonucleotide is modified as described in one of the modifications above. The modification can either cover the oligonucleotide continuously or irregularly.

In yet another embodiment, at least two modifications as described above are combined within one oligonucleotide. In another embodiment, 1 to about 12 or 1 to about 8 or 1 to about 4 or 1 to about 2 oligonucleotides and/or nucleotide linkages at the 3 ' and/or 5 'end of the oligonucleotide are modified as described above.

In a preferred embodiment, an oligonucleotide is selected from the group consisting of an antisense oligonucleotide, siRNA, RNAi, and an aptamer, or a combination thereof.

In one embodiment, the oligodeoxynucleotides are used neat or in the form of a pharmaceutically acceptable salt. The salts have to be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic acid. Also, such salts can be prepared as alkaline metal or alkaline earth salts, such as sodium, potassium or calcium salts of the carboxylic acid group.

In another embodiment, the pharmaceutical composition further comprises at least one buffer and/or at least one preservative. In one embodiment suitable buffering agents include but are not limited to acetic acid and a salt (e.g., 1-2% w/v), citric acid and a salt (e.g., 1-3% w/v), boric acid and a salt (e.g., 0.5-2.5% w/v), and phosphoric acid and a salt (e.g., 0.8-2% w/v). Suitable preservatives include benzalkonium chloride (e.g., 0.003-0.03% w/v), chlorobutanol (e.g., 0.3-0.9% w/v), parabens (e.g., 0.01-0.25% w/v), and thiomersal (e.g., 0.004-0.02% w/v).

In yet another embodiment the pharmaceutical compositions also include penetration enhancers in order to enhance the alimentary delivery. Penetration enhancers may be classified as belonging to one of five broad categories, i.e., fatty acids, bile salts, chelating agents, surfactants, and non-surfactants (Lee et al., 1991; Muranishi, 1990). One or more penetration enhancers from one or more of these broad categories may be included.

Various fatty acids and their derivatives, which act as penetration enhancers, include, for example, oleic acid, lauric acid, capric acid, myristic acid, palmitic acid, stearic acid, linoleic acid, linolenic acid, dicaprate, tricaprate, recinleate, monoolein (1-monooleoyl-rac-glycerol), dilaurin, caprylic acid, araichidonic acid, glyceryl 1 -monocaprate, 1-dodecylazacycloheptan-2-one, acylcarnitines, acylcholines, mono- and di-glycerides and physiologically acceptable salts thereof (i.e., oleate, laurate, caprate, myristate, palmitate, stearate, linoleate, etc.) (Lee et al., 1991; Muranishi, 1990; El-Hariri et al., 1992). Examples of some presently preferred fatty acids are sodium caprate and sodium laurate, used singly or in combination at concentrations of for example 0.5 to 5%.

The physiological roles of bile include the facilitation of dispersion and absorption of lipids and fat-soluble vitamins (Brunton, 1996). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), used in concentrations of for example 0.5 to 2%.

Complex formulations comprising one or more penetration enhancers may be used. For example, bile salts may be used in combination with fatty acids to produce complex formulations. Preferred combinations include CDCA combined with sodium caprate or sodium laurate (e.g., 0.5 to 5%).

In one embodiment, additionally chelating agents are used that include, but are not limited to, disodium ethylenediaminetetraacetate (EDTA), citric acid, salicylates (e.g., sodium salicylate, 5-methoxysalicylate and homovanillate), N-acyl derivatives of collagen, laureth-9 and N-amino acyl derivatives of beta-diketones (enamines) (Lee et al., 1991 ; Muranishi, 1990; Buur et al. 1990). Chelating agents have the added advantage of also serving as DNase inhibitors. In yet another embodiment additionally surfactants are used. Surfactants include, for example, sodium lauryl sulphate, polyoxyethylene-9-lauryl ether and polyoxyethylene- 20-cetyl ether (Lee et al., 1991), and perfluorochemical emulsions such as FC-43 (Takahashi et al., 1988). Non-surfactants include, for example, unsaturated cyclic ureas, 1-alkyl- and 1- alkenylazacyclo-alkanone derivatives (Lee et al., 1991), and non-steroidal antiinflammatory agents such as diclofenac sodium, indomethacin, and phenylbutazone (Yamashita et al., 1987). In one embodiment, the pharmaceutical composition of the present invention additionally contains other adjunct components conventionally found in pharmaceutical compositions, at their art-established usage levels. Thus, for example, the compositions may contain additional compatible pharmaceutically active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents, and stabilizers. However, such materials, when added, should not unduly interfere with the biological activities of the components of the compositions of the invention.

The present invention is further directed to a pharmaceutical composition comprising an oligonucleotide for use in prophylaxis and/or treatment of a TGF-beta1 and TGF-beta 2 overexpressing tumor, a TGF-beta2 and TGF-beta3 overexpressing tumor, a TGF-beta1 and TGF-beta3 overexpressing tumor. Optionally, the pharmaceutical composition comprises a pharmaceutically acceptable carrier, filer lubricant, diluent, excipient, disintegrate, and/or adjuvant.

In a preferred embodiment, the pharmaceutical composition is administered orally, intravenously, intracranially, intraperitoneally, intravesically, parenterally, topically, transdermally, subconjunctivally, or sublingually.

### Examples

The following examples show the present invention in more detail, but do not limit the invention to the examples.

### Example 1

### Inhibition of TGF-beta1 and TGF-beta2 mRNA overexpression

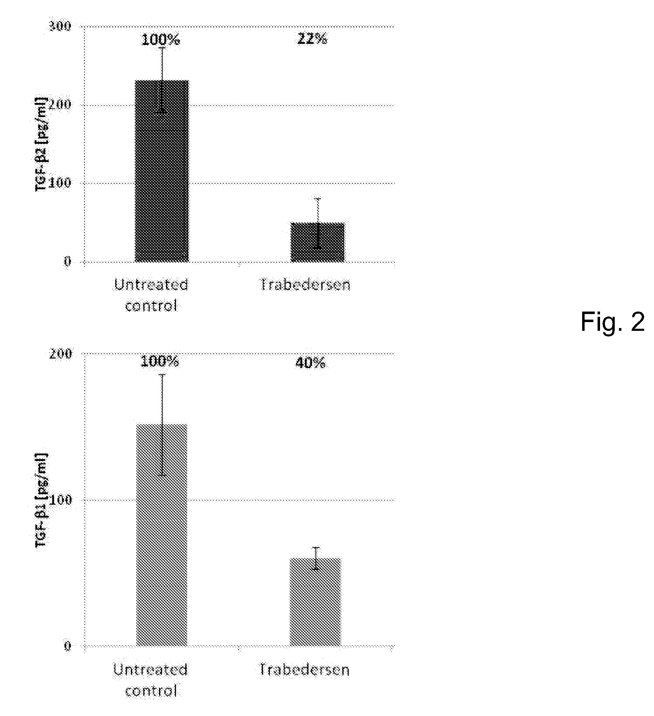

A-172 human glioblastoma cells were treated with 5 µM oligonucleotide of SEQ ID No.30 for a total treatment time of 7d with FBS either absent or present during the last 3 d of treatment. TGF-beta1 and TGF-beta2 protein levels in cell supernatants were determined by ELISA and are presented relative to untreated control (100%). Results are shown in Fig. 2.

The oligonucleotide of SEQ ID No. 30 reduced the significantly the expression of TGF-beta1 and TGF-beta2 in A-172 cells.

## Claims

1. Oligonucleotide for use in prophylaxis and/or treatment of a TGF-beta1 and TGF-beta 2 mRNA overexpressing tumor, a TGF-beta2 and TGF-beta3 mRNA overexpressing tumor, a TGF-beta1 and TGF-beta3 mRNA overexpressing tumor, or a TGF-beta1, TGF-beta 2, and TGF-beta3 mRNA overexpressing tumor.

2. Oligonucleotide according to claim 1, wherein the oligonucleotide is selected from the group consisting of an antisense oligonucleotide, siRNA, RNAi, and an aptamer, or a combination thereof.

3. Oligonucleotide according to claim 1 or 2, wherein the antisense oligonucleotide hybridizes with mRNA of TGF-beta1, TGF-beta-2, or TGF-beta3.

4. Oligonucleotide according to any of claims 1 to 3, wherein the antisense oligonucleotide is selected from the group consisting of SEQ ID No. 1 to 78.

5. Oligonucleotide according to any of claims 1 to 4, wherein the tumor is selected from the group consisting of glioma, astrocytoma, pancreatic carcinoma, prostate carcinoma, lung carcinoma, skin cancer, kidney cancer, renal carcinoma, ovary cancer, breast cancer, esophageal cancer, stomach cancer, bladder cancer, colon cancer, colorectal cancer, gastric cancer, nasopharynx or larynx cancer, thyreoid gland cancer, bone cancer, pleura cancer, liver cancer, uterine cancer, endometrial cancer, carcinoma embronale, choriocarcinoma, teratoma, Hodgkin's Lymphoma (NHL), or leukemia.

6. Oligonucleotide according to any of claims 1 to 5, wherein the tumor is selected from the group consisting of disseminated melanoma, malignant melanoma, glioblastoma multiforme, astrocytoma malignum, ductal adenocarcinoma, non-small cell lung carcinoma (NSCLC), squamous cell lung cancer, adenocarcinoma, large cell lung cancer, small cell lung cancer (SCLC)), squamous cell carcinoma, basal cell carcinoma, renal cell carcinoma, pheochromocytoma, urothelial cell carcinoma, adenocarcinoma, ovarian epithelial carcinoma, mucinous cystadenocarcinoma, ductal carcinoma, renal clear cell carcinoma, lobular carcinoma, squamous epithelium carcinoma, colorectal cancer, gastric cancer, lymphoepithelioma, papillary thyroid cancer, follicular thyroid cancer, medullary thyroid cancer, anaplastic thyreoid cancer, osteosarcoma, chondrosarcoma, mesothelioma, heptocellular carcinoma, acute or myeloblastic leukemia.

7. Oligonucleotide according to any of claims 1 to 6, wherein the tumor is equivalent to PC-3 cells (prostate), A-549 cells (NSCLC), pediatric glioma, PANC-1 cells (pancreas), PA-TU-8902 cells (pancreas), L3.6pL cells (pancreas), CAKI (renal cell), DU-145 (prostate), Mel-Juso (melanoma), C8161 (melanoma), KYSE-140 (esophagus), A-172 (glioma), or NCI-H661 (lung).

8. Pharmaceutical composition comprising an oligonucleotide according to any of claims 1 to 7 for use in prophylaxis and/or treatment of a TGF-beta1 and TGF-beta 2 overexpressing tumor, a TGF-beta2 and TGF-beta3 overexpressing tumor, a TGF-beta1 and TGF-beta3 overexpressing tumor.

9. Pharmaceutical composition according to claim 8 comprising a pharmaceutically acceptable carrier, filer lubricant, diluent, excipient, disintegrate, and/or adjuvant.

10. Pharmaceutical composition according to claim 8 or 9, wherein the pharmaceutical composition is administered orally, intravenously, intracranially, intraperitoneally, intravesically, parenterally, topically, transdermally, subconjunctivally, or sublingually.

11. Method for the preparation of a pharmaceutical composition according to any of claims 8 to 10.


SEQUENCE LISTING

<110>  Antisense Pharma GmbH

<120>  Oligonucleotides for use in prophylaxis and/or treatment of
       TGF-beta1 and TGF-beta2, TGF-beta2 and TGF-beta3, TGF-beta1 and
       TGF-beta3, or TGF-beta1, TGF-beta2, and TGF-beta3 mRNA
       overexpressing diseases

<130>  P93239EP00

<140>  EP 10191103.0
<141>  2010-11-12

<160>  78

<170>  PatentIn version 3.3

<210>  1
<211>  14
<212>  DNA
<213>  Artificial

<220>
<223>  antisense oligonucleotide TGF beta 1

<400>  1
cgatagtctt gcag                                                    14


<210>  2
<211>  14
<212>  DNA
<213>  Artificial

<220>
<223>  antisense oligonucleotide TGF beta 1

<400>  2
gtcgatagtc ttgc                                                    14


<210>  3
<211>  14
<212>  DNA
<213>  Artificial

<220>
<223>  antisense oligonucleotide TGF beta 1

<400>  3
cttggacagg atct                                                    14


<210>  4
<211>  14
<212>  DNA
<213>  Artificial

<220>
<223>  antisense oligonucleotide TGF beta 1

<400>  4
ccaggaattg ttgc                                                    14


<210>  5
<211>  14

<212>  DNA
<213>  Artificial

<220>
<223>  antisense oligonucleotide TGF beta 1

<400>  5
cctcaatttc ccct                                                    14


<210>  6
<211>  14
<212>  DNA
<213>  Artificial

<220>
<223>  antisense oligonucleotide TGF beta 1

<400>  6
gatgtccact tgca                                                    14


<210>  7
<211>  14
<212>  DNA
<213>  Artificial

<220>
<223>  antisense oligonucleotide TGF beta 1

<400>  7
ctccaaatgt aggg                                                    14


<210>  8
<211>  14
<212>  DNA
<213>  Artificial

<220>
<223>  antisense oligonucleotide TGF beta 1

<400>  8
accttgctgt actg                                                    14


<210>  9
<211>  14
<212>  DNA
<213>  Artificial

<220>
<223>  antisense oligonucleotide TGF beta 1

<400>  9
gtagtacacg atgg                                                    14


<210>  10
<211>  14
<212>  DNA
<213>  Artificial

<220>
<223>  antisense oligonucleotide TGF beta 1

<400>  10
cacgtagtac acga                                                    14

<210>  11
<211>  14
<212>  DNA
<213>  Artificial

<220>
<223>  antisense oligonucleotide TGF beta 1

<400>  11
catgttggac agct                                                          14


<210>  12
<211>  14
<212>  DNA
<213>  Artificial

<220>
<223>  antisense oligonucleotide TGF beta 1

<400>  12
gcacgatcat gttg                                                          14


<210>  13
<211>  16
<212>  DNA
<213>  Artificial

<220>
<223>  antisense oligonucleotide TGF beta 1

<400>  13
tgtactctgc ttgaac                                                        16


<210>  14
<211>  18
<212>  DNA
<213>  Artificial

<220>
<223>  antisense oligonucleotide TGF beta 1

<400>  14
ctgatgtgtt gaagaaca                                                      18


<210>  15
<211>  16
<212>  DNA
<213>  Artificial

<220>
<223>  antisense oligonucleotide TGF beta 1

<400>  15
ctctgatgtg ttgaag                                                        16


<210>  16
<211>  16
<212>  DNA
<213>  Artificial

<220>

<223>  antisense oligonucleotide TGF beta 1

<400>  16
ggaagtcaat gtacag                                                        16


<210>  17
<211>  18
<212>  DNA
<213>  Artificial

<220>
<223>  antisense oligonucleotide TGF beta 1

<400>  17
catgtcgata gtcttgca                                                      18


<210>  18
<211>  18
<212>  DNA
<213>  Artificial

<220>
<223>  antisense oligonucleotide TGF beta 1

<400>  18
agctgaagca atagttgg                                                      18


<210>  19
<211>  18
<212>  DNA
<213>  Artificial

<220>
<223>  antisense oligonucleotide TGF beta 1

<400>  19
gtcatagatt tcgttgtg                                                      18


<210>  20
<211>  18
<212>  DNA
<213>  Artificial

<220>
<223>  antisense oligonucleotide TGF beta 1

<400>  20
ctccactttt aacttgag                                                      18


<210>  21
<211>  18
<212>  DNA
<213>  Artificial

<220>
<223>  antisense oligonucleotide TGF beta 1

<400>  21
tgctgtattt ctggtaca                                                      18


<210>  22
<211>  14

<212>  DNA
<213>  Artificial

<220>
<223>  antisense oligonucleotide TGF beta 2

<400>  22
cacacagtag tgca                                                          14


<210>  23
<211>  14
<212>  DNA
<213>  Artificial

<220>
<223>  antisense oligonucleotide TGF beta 2

<400>  23
gcacacagta gtgc                                                          14


<210>  24
<211>  17
<212>  DNA
<213>  Artificial

<220>
<223>  antisense oligonucleotide TGF beta 2

<400>  24
gcttgctcag gatctgc                                                       17


<210>  25
<211>  14
<212>  DNA
<213>  Artificial

<220>
<223>  antisense oligonucleotide TGF beta 2

<400>  25
tactcttcgt cgct                                                          14


<210>  26
<211>  14
<212>  DNA
<213>  Artificial

<220>
<223>  antisense oligonucleotide TGF beta 2

<400>  26
cttggcgtag tact                                                          14


<210>  27
<211>  14
<212>  DNA
<213>  Artificial

<220>
<223>  antisense oligonucleotide TGF beta 2

<400>  27
gtaaacctcc ttgg                                                          14

<210>  28
<211>  19
<212>  DNA
<213>  Artificial

<220>
<223>  antisense oligonucleotide TGF beta 2

<400>  28
gtctattttg taaacctcc                                                    19


<210>  29
<211>  20
<212>  DNA
<213>  Artificial

<220>
<223>  antisense oligonucleotide TGF beta 2

<400>  29
gcatgtctat tttgtaaacc                                                   20


<210>  30
<211>  18
<212>  DNA
<213>  Artificial

<220>
<223>  antisense oligonucleotide TGF beta 2

<400>  30
cggcatgtct attttgta                                                     18


<210>  31
<211>  14
<212>  DNA
<213>  Artificial

<220>
<223>  antisense oligonucleotide TGF beta 2

<400>  31
ggcatcaagg tacc                                                         14


<210>  32
<211>  14
<212>  DNA
<213>  Artificial

<220>
<223>  antisense oligonucleotide TGF beta 2

<400>  32
ctgtagaaag tggg                                                         14


<210>  33
<211>  18
<212>  DNA
<213>  Artificial

<220>

<223>  antisense oligonucleotide TGF beta 2

<400>  33
acaattctga agtagggt                                                    18


<210>  34
<211>  18
<212>  DNA
<213>  Artificial

<220>
<223>  antisense oligonucleotide TGF beta 2

<400>  34
tcaccaaatt ggaagcat                                                    18


<210>  35
<211>  20
<212>  DNA
<213>  Artificial

<220>
<223>  antisense oligonucleotide TGF beta 2

<400>  35
gctttcacca aattggaagc                                                  20


<210>  36
<211>  14
<212>  DNA
<213>  Artificial

<220>
<223>  antisense oligonucleotide TGF beta 2

<400>  36
ctggcttttg ggtt                                                        14


<210>  37
<211>  18
<212>  DNA
<213>  Artificial

<220>
<223>  antisense oligonucleotide TGF beta 2

<400>  37
tctgatatag ctcaatcc                                                    18


<210>  38
<211>  18
<212>  DNA
<213>  Artificial

<220>
<223>  antisense oligonucleotide TGF beta 2

<400>  38
tcctagtgga ctttatag                                                    18


<210>  39
<211>  16

<212>  DNA
<213>  Artificial

<220>
<223>  antisense oligonucleotide TGF beta 2

<400>  39
tttttcctag tggact                                                        16


<210>  40
<211>  19
<212>  DNA
<213>  Artificial

<220>
<223>  antisense oligonucleotide TGF beta 2

<400>  40
caattatcct gcacatttc                                                     19


<210>  41
<211>  16
<212>  DNA
<213>  Artificial

<220>
<223>  antisense oligonucleotide TGF beta 2

<400>  41
gcaattatcc tgcaca                                                        16


<210>  42
<211>  16
<212>  DNA
<213>  Artificial

<220>
<223>  antisense oligonucleotide TGF beta 2

<400>  42
gcagcaatta tcctgc                                                        16


<210>  43
<211>  14
<212>  DNA
<213>  Artificial

<220>
<223>  antisense oligonucleotide TGF beta 2

<400>  43
tggcattgta ccct                                                          14


<210>  44
<211>  14
<212>  DNA
<213>  Artificial

<220>
<223>  antisense oligonucleotide TGF beta 2

<400>  44
tgtgctgagt gtct                                                          14

<210>  45
<211>  16
<212>  DNA
<213>  Artificial

<220>
<223>  antisense oligonucleotide TGF beta 2

<400>  45
cctgctgtgc tgagtg                                                    16


<210>  46
<211>  14
<212>  DNA
<213>  Artificial

<220>
<223>  antisense oligonucleotide TGF beta 2

<400>  46
cttgggtgtt ttgc                                                      14


<210>  47
<211>  18
<212>  DNA
<213>  Artificial

<220>
<223>  antisense oligonucleotide TGF beta 2

<400>  47
tttagctgca tttgcaag                                                  18


<210>  48
<211>  14
<212>  DNA
<213>  Artificial

<220>
<223>  antisense oligonucleotide TGF beta 2

<400>  48
gccacttttc caag                                                      14


<210>  49
<211>  14
<212>  DNA
<213>  Artificial

<220>
<223>  antisense oligonucleotide TGF beta 3

<400>  49
tcgagcttcc ccca                                                      14


<210>  50
<211>  14
<212>  DNA
<213>  Artificial

<220>

<223>  antisense oligonucleotide TGF beta 3

<400>  50
ccccgagccc aagg                                                          14


<210>  51
<211>  13
<212>  DNA
<213>  Artificial

<220>
<223>  antisense oligonucleotide TGF beta 3

<400>  51
cccgacgagc cgg                                                           13


<210>  52
<211>  14
<212>  DNA
<213>  Artificial

<220>
<223>  antisense oligonucleotide TGF beta 3

<400>  52
acgcaccaag gcga                                                          14


<210>  53
<211>  15
<212>  DNA
<213>  Artificial

<220>
<223>  antisense oligonucleotide TGF beta 3

<400>  53
cgggttgtcg agccc                                                         15


<210>  54
<211>  13
<212>  DNA
<213>  Artificial

<220>
<223>  antisense oligonucleotide TGF beta 3

<400>  54
cggcagtgcc ccg                                                           13


<210>  55
<211>  14
<212>  DNA
<213>  Artificial

<220>
<223>  antisense oligonucleotide TGF beta 3

<400>  55
cgcaattctg ctcg                                                          14


<210>  56
<211>  14

<212>  DNA
<213>  Artificial

<220>
<223>  antisense oligonucleotide TGF beta 3

<400>  56
ttcgttgtgc tccc                                                          14


<210>  57
<211>  14
<212>  DNA
<213>  Artificial

<220>
<223>  antisense oligonucleotide TGF beta 3

<400>  57
attccgactc ggtg                                                          14


<210>  58
<211>  17
<212>  DNA
<213>  Artificial

<220>
<223>  antisense oligonucleotide TGF beta 3

<400>  58
acgtgcgtca tcaccgt                                                       17


<210>  59
<211>  10
<212>  DNA
<213>  Artificial

<220>
<223>  antisense oligonucleotide TGF beta 3

<400>  59
ccaagaagcc                                                               10


<210>  60
<211>  14
<212>  DNA
<213>  Artificial

<220>
<223>  antisense oligonucleotide TGF beta 3

<400>  60
cctaatgcct tcca                                                          14


<210>  61
<211>  14
<212>  DNA
<213>  Artificial

<220>
<223>  antisense oligonucleotide TGF beta 3

<400>  61
tcagcagggc cagg                                                          14

<210> 62
<211> 17
<212> DNA
<213> Artificial

<220>
<223> antisense oligonucleotide TGF beta 3

<400> 62
gcaaagttca gcagggc 17


<210> 63
<211> 16
<212> DNA
<213> Artificial

<220>
<223> antisense oligonucleotide TGF beta 3

<400> 63
ggcaaagttc agcagg 16


<210> 64
<211> 18
<212> DNA
<213> Artificial

<220>
<223> antisense oligonucleotide TGF beta 3

<400> 64
gtggcaaagt tcagcagg 18


<210> 65
<211> 14
<212> DNA
<213> Artificial

<220>
<223> antisense oligonucleotide TGF beta 3

<400> 65
gtggcaaagt tcag 14


<210> 66
<211> 18
<212> DNA
<213> Artificial

<220>
<223> antisense oligonucleotide TGF beta 3

<400> 66
gaccgtggca aagttcag 18


<210> 67
<211> 15
<212> DNA
<213> Artificial

<220>

<223>  antisense oligonucleotide TGF beta 3

<400>  67
agagaggctg accgt                                                        15


<210>  68
<211>  17
<212>  DNA
<213>  Artificial

<220>
<223>  antisense oligonucleotide TGF beta 3

<400>  68
gagagagaga ggctgac                                                      17


<210>  69
<211>  15
<212>  DNA
<213>  Artificial

<220>
<223>  antisense oligonucleotide TGF beta 3

<400>  69
acagagagag gctga                                                        15


<210>  70
<211>  15
<212>  DNA
<213>  Artificial

<220>
<223>  antisense oligonucleotide TGF beta 3

<400>  70
gtggacagag agagg                                                        15


<210>  71
<211>  18
<212>  DNA
<213>  Artificial

<220>
<223>  antisense oligonucleotide TGF beta 3

<400>  71
caactggaca gagagagg                                                     18


<210>  72
<211>  16
<212>  DNA
<213>  Artificial

<220>
<223>  antisense oligonucleotide TGF beta 3

<400>  72
tcttcttgat gtggcc                                                       16


<210>  73
<211>  17

<212>  DNA
<213>  Artificial

<220>
<223>  antisense oligonucleotide TGF beta 3

<400>  73
ccctcttctt cttgatg                                                    17


<210>  74
<211>  14
<212>  DNA
<213>  Artificial

<220>
<223>  antisense oligonucleotide TGF beta 3

<400>  74
caccctcttc ttct                                                       14


<210>  75
<211>  17
<212>  DNA
<213>  Artificial

<220>
<223>  antisense oligonucleotide TGF beta 3

<400>  75
atggatttct ttggcat                                                    17


<210>  76
<211>  13
<212>  DNA
<213>  Artificial

<220>
<223>  antisense oligonucleotide TGF beta 3

<400>  76
ggatttcttt ggc                                                        13


<210>  77
<211>  18
<212>  DNA
<213>  Artificial

<220>
<223>  antisense oligonucleotide TGF beta 3

<400>  77
aagttggact ctcttctc                                                   18


<210>  78
<211>  18
<212>  DNA
<213>  Artificial

<220>
<223>  antisense oligonucleotide TGF beta 3

<400>  78
taagttggac tctcttct                                                   18